# EUROPEAN PATENT APPLICATION

(11) **EP 4 664 179 A1**
(43) Date of publication of application: **17.12.2025**
(21) Application number: 24181432.6
(22) Date of filing: 11.06.2024
(51) Int. Cl.: G02B 21/00, G02B 21/24, G02B 7/00

(54) **HOUSING FOR A MICROSCOPE, MICROSCOPE AND OPTICAL IMAGING SYSTEM**

(71) Applicant: Leica Instruments (Singapore) Pte. Ltd., Singapore 618299 (SG)
(72) Inventor: SZABO, Patrick, 618299 Singapore (SG)
(74) Representative: 2SPL Patentanwälte PartG mbB

(57) **Abstract**

A housing is provided. The housing comprises an outer shell and a tracer arranged in the outer shell. The outer shell forms a cavity for arranging an optical component of the microscope.

## Description

### Technical field

Examples relate to a housing for a microscope, a microscope and an optical imaging system.

### Background

In image-guided surgery (IGS) measurements a tracker device, often referred to as a navigation or tracking system, is used to precisely track the position and orientation of the surgical instruments or the microscope itself in real-time. This information can then be integrated with preoperative imaging data such as Magnetic Resonance Imaging (MRI) or Computed Tomography (CT) scans to provide enhanced guidance to the surgeon during the procedure, ensuring greater accuracy and safety. However, an arrangement of the tracker device, e.g., a tracer part of the tracker device, may depend on several factors.

Thus, there may be a need to improve a microscope system comprising a tracer or a tracker device, e.g., for an IGS measurement.

### Summary

It is a finding of the inventors that a tracer can be included in the housing of a microscope. That is, a tracer can no longer be attached to the microscope of the optical imaging system, rather it can be integrated into the actual microscope, i.e., the housing of the microscope. In this way, an arrangement of the tracer can be facilitated.

Examples provide a housing for a microscope. The housing comprises an outer shell and a tracer arranged in the outer shell. The outer shell forms a cavity for arranging an optical component of the microscope. Including the tracer in the outer shell of the housing may allow to integrate the tracer into the microscope. In this way, attaching a tracer to the microscope can be avoided. Thus, an influence of the tracer on the microscope can be reduced. For example, this may prevent a balancing of the microscope from being affected by the tracer.

In an example, the tracer may be arranged in a side wall of the outer shell. Thus, the tracer can be easily observed from a side of the microscope. In this way, the detectability of the tracer can be improved.

In an example, the side wall may have a wall surface. A wall surface, also referred to as enclosure surface, may be a surface that encloses or surrounds a space. For example, a wall surface may comprise curved and/or flat sections of a side wall (or multiple side walls). That is, the wall surface may form a boundary of a structure or space. Thus, the wall surface is different from an external tracer which is attached to the microscope.

In an example, the housing may further comprise a plurality of tracers arranged in a plurality of side walls of the outer shell. In this way, a position of the microscope can be determined from multiple directions. Further, a detection of the position of the microscope can be improved by the plurality of tracers.

In an example, a further tracer of the plurality of tracers may be arranged in a second side wall adjacent to the side wall. In this way, the position of the microscope can be determined in a facilitated way. Further, a reliability of the detection of the position of the microscope can be increased.

In an example, an opposing tracer of the plurality of tracers may be arranged in an opposing side wall opposite the side wall. In this way, the position of the microscope can be determined from opposite angles.

In an example, the tracer may comprise a light source and/or a reflective marker. Thus, the tracer can be easily integrated into the housing.

In an example, the tracer may be configured to allow a determination of a position of the housing in three dimensions. For example, the tracer may comprise three light sources, which allow a determination of a position of the tracer.

In an example, the cavity is completely surrounded by the outer shell. That is, the cavity may be an enclosed space within the housing, to accommodate internal components.

Examples provide a microscope comprising a housing as described above and an optical component. The optical component is housed by the housing. In an example, the microscope may be a surgical microscope.

Examples provide an optical imaging system comprising a housing as described above or a microscope as described above.

### Short description of the Figures

Some examples of apparatuses and/or methods will be described in the following by way of example only, and with reference to the accompanying figures, in which
Fig. 1 shows a housing for a microscope;
Fig. 2 shows an example of an optical imaging system comprising a housing for a microscope as described with reference to Fig. 1; and
Fig. 3 shows a schematic illustration of a system.

### Detailed Description

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Various examples will now be described more fully with reference to the accompanying drawings in which some examples are illustrated. In the figures, the thicknesses of lines, layers and/or regions may be exaggerated for clarity.

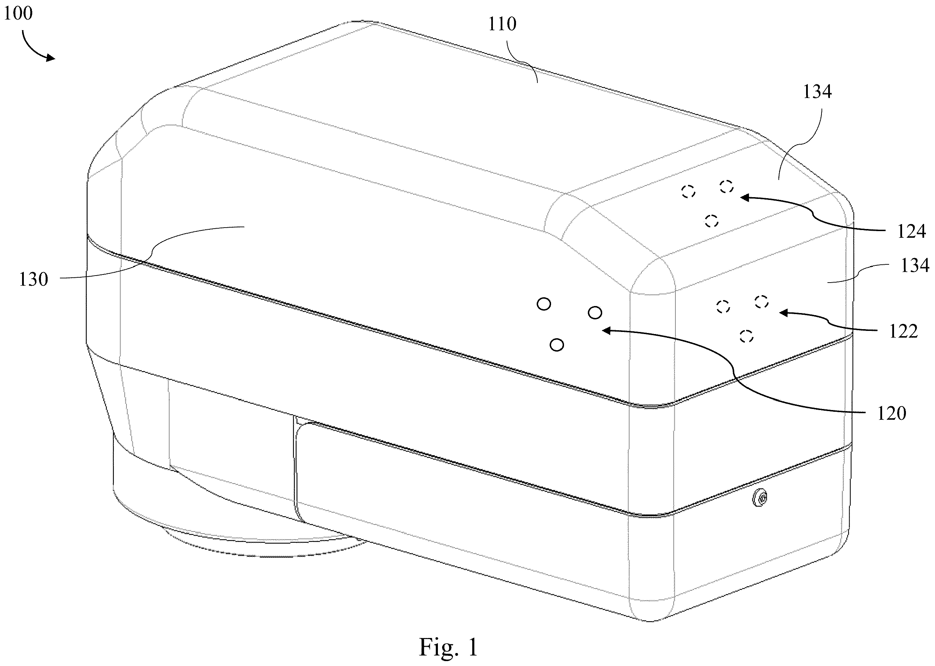

Fig. 1 shows a housing 100 for a microscope. The housing 100 comprises an outer shell 110 and a tracer 120 arranged in the outer shell 110. The outer shell 110 forms a cavity for arranging an optical component of the microscope.

The cavity may be essentially an enclosed space within the housing 100, designed to accommodate various internal components such like optical components. For example, in the context of a surgical microscope, the housing 100 may typically enclose a cavity where optical components, such as lenses, prisms, and mirrors, are housed. The cavity may provide a protected environment for these delicate components, shielding them from external contaminants and physical damage while allowing them to function effectively. That is, the housing 100 can be a carrier for the optic of the microscope.

Optionally, the cavity within the housing 100 may also house mechanical components, such as motors and gears, used for adjusting magnification, focus, and/or positioning of the optical components. It may be required to house these mechanical components within the cavity to ensure proper functionality and alignment of the microscope. That is, the cavity within the housing 100 may serve as a critical space for housing 100 and protecting the internal components of the microscope, contributing to its overall functionality and durability.

The cavity within the housing 100 does not need to be always fully enclosed. While cavities are typically enclosed spaces within the housing 100, there can be variations depending on the design and function of the housing 100 (or optionally the microscope). In some cases, the cavity may have openings or access points for various purposes, e.g., an access port, a ventilation opening, a cable passage, an optical path. Overall, while cavities within housings are typically enclosed to protect internal components, there can be various openings or access points designed into the housing 100 for specific functional or operational requirements.

The outer shell 110 may be made or comprise metal and/or plastic, providing structural integrity and protection against damage. It may serve as the (main) body of the microscope, enclosing the optical components, and optionally mechanical components. The outer shell 110 of the housing 100 my play a crucial role in ensuring the functionality, durability, and user-friendliness of the microscope.

The outer shell 110 may be an integral part of the structure of the microscope, e.g., forming its primary body or framework. It may have features like ports or attachment points designed to accommodate external devices (e.g., an external tracker device comprising an external tracer known in the prior art), but these external devices are not physically integrated into the outer shell 110 itself. In contrast, an external tracker device is physically separate from the outer shell 110 and a part of the external tracker device, e.g., an external tracer, is attached to it externally, often through mounting brackets, connectors, or other attachment mechanisms. That is, the outer shell 110 may be a permanent and integral part of the microscope, an external tracker device comprising an external tracer may be removable or detachable. An external tracker device allows for flexibility in usage and customization, as the external tracker device can be attached or removed as needed for different applications or scenarios. However, an external tracker device add weight to the microscope, which could have a negative impact on balancing and draping. It is a finding of the inventors, that a microscope used for IGS measurements can be improved by including the tracer in the housing 100 of the microscope. In this way, a negative impact of an external tracker device, e.g., the tracer part of the external tracker, on the microscope can be avoided. Further, the tracer part of the housing 100 can provide an industry standard, such that it can be used independently of a specific supplier of IGS measurement equipment. The tracer 120 may be integrated in the microscope body, i.e., the housing 100. The tracer 120 may comprise a defined mechanical pattern. For example, a plurality of tracers may be arranged on several sides of the housing 100.

In an example, the tracer 120 may be arranged in a side wall 130 of the outer shell 110. For example, the tracer 120 may be integrated into the side wall 130 of the outer shell 110. That is, the tracer 120 can be detected from a specific angle. A side wall may refer to a vertical or a lateral surface that forms a side of the outer shell 110. For example, a rectangular outer shell can have four vertical surfaces and two lateral surfaces that run parallel to each other, which are considered side walls. These side walls enclose the cavity formed by the outer shell 110. The side wall 130 of the outer shell 110 can vary depending on the shape and design of the outer shell 110 or the housing 100 itself. The side wall 130 may provide enclosure and/or protection for the internal components of the microscope within the outer shell 110. The form of the housing 100 in Fig. 1 is only an example. For example, the housing 100 may have an alternative cylindrical shape. In the case of a cylindrical outer shell 110 (or housing 100), the curved surface that wraps around the circumference of the outer shell 110 would be considered the side wall 130.

In an example, the side wall 130 may have a wall surface. A wall surface, also referred to as enclosure surface may be a surface that encloses or surrounds a space. A wall surface may refer to any continuous surface that forms part of the enclosing structure of the housing 100. This wall surface may be flat, curved, angled, or irregular in shape, depending on the design and construction of the housing 100. A wall surface can exhibit a variety of shapes and configurations to accommodate functional requirements.

In an example, the housing 100 may further comprise a plurality of tracers 120, 122, 124 arranged in a plurality of side walls 130, 132, 134 of the outer shell 110. That is, the plurality of tracers 120, 122, 124 can be detected from different angles, i.e., from different positions relative to the housing 100. Thus, in comparison to an external tracker device, which is attached to the housing 100, the plurality of tracers 120, 122, 124 may improve the possibility to detect the position of the housing 100 from multiple directions. The position of the housing 100 may comprise a location of the housing 100 and/or an orientation of the housing 100. That is, the position of the housing 100 may define the accurate alignment of the housing 100 in the space, e.g., in a surgery room.

In an example, a further tracer 122, 124 of the plurality of tracers 120, 122, 124 may be arranged in a second side wall 132, 134 adjacent to the side wall 130. That is, different tracer 120, 122, 124 can be integrated into different adjacent side walls 130, 132, 134. In this way, a determination of the position of the housing 100 can be improved. Further, even when the tracer 120, cannot be detected, e.g., a view of a camera on the tracer 120 is obstructed by the surgeon, the further tracer 122, 124 can be used to determine the position of the housing 100. In an example, an opposing tracer (not shown) of the plurality of tracers 120, 122, 124 may be arranged in an opposing side wall (not shown) opposite the side wall 130. In this way, the position of the microscope can be determined from opposite angles. Thus, a reliability of the determination of the position of the housing 100 can be improved by the plurality of tracers 120, 122, 124.

In an example, the tracer 120 may comprise a light source and/or a reflective marker. Thus, the tracer can be easily integrated into the housing 100. For example, the tracer 120 can be a light source emitting a specific wavelength of light, e.g., fluorescence light. That is, the tracer 120 can actively radiate light. The light emitted from the tracer 120 can be detected by a detection device, e.g., a camera (external to or part of an optical imaging system). In this case, the light source can be controlled by an apparatus part of the microscope or an external apparatus, e.g., part of the camera or an external IGS measurement system. For example, the light source can be controlled so that it is switched on or off or has a flashing frequency.

Optionally or alternatively, the tracer 120 may reflect light emitted from an external light source, e.g., part of an external IGS measurement system or part of the optical imaging system. In this case, the tracer 120 may only reflect the light. That is, the tracer 120 may not actively radiate light. The tracer 120 may be comprise passive reflective markers configured to reflect light. The IGS measurement system may detect the emitted or reflected light and uses it to precisely monitor position of the tracer 120 in real-time during a surgery. This tracking information can then be integrated into a surgical navigation software to provide accurate guidance to the surgeon based on preoperative imaging data, for example.

In an example, the tracer 120 may be configured to allow a determination of a position of the housing 100 in three dimensions. For example, as can be seen in Fig. 1, the tracer 120 may comprise three different elements, e.g., light sources and/or reflective markers. The geometry of the three different elements may be known, such that the position of the three different elements can be used to determine the position, i.e., the location and/or the orientation, of the tracer 120. For example, the different elements may have a defined mechanical pattern. That is, the three different elements arranged with the defined mechanical pattern may define the tracer 120. The three different elements are for illustrative purposes only. Other mechanical patterns or more elements may be used to define a tracer 120, 122, 124. Multiple tracers of the plurality of tracers 120, 122, 124 may be identical. Optionally or alternatively, a tracer 120, 122, 124 may be adapted to fit a side wall 130, 132, 134. The description for the tracer 120 may also apply for other tracers of the plurality of tracers 120, 122, 124.

The tracer 120 may comprise sensors, (reflective) markers, or other tracing elements that are integrated into the housing 100 itself. The tracer 120 may communicate with an IGS measurement system, which processes data and provides continuous spatial information to a surgical navigation software. The primary function of the tracer 120 may be to enable precise navigation and guidance for the surgeon, ensuring accurate targeting and manipulation of visualization of anatomical structures based on preoperative imaging data. The IGS measurement system may be part of the optical imaging system comprising the microscope or may be external. The surgical navigation software may be executed on an apparatus of the optical imaging system, or an apparatus of the IGS measurement system. That is, the tracer 120 may be controlled by the optical imaging system or the IGS measurement system.

In an example, the cavity is completely surrounded by the outer shell 110. That is, the cavity may be an enclosed space within the housing 100, to accommodate internal components.

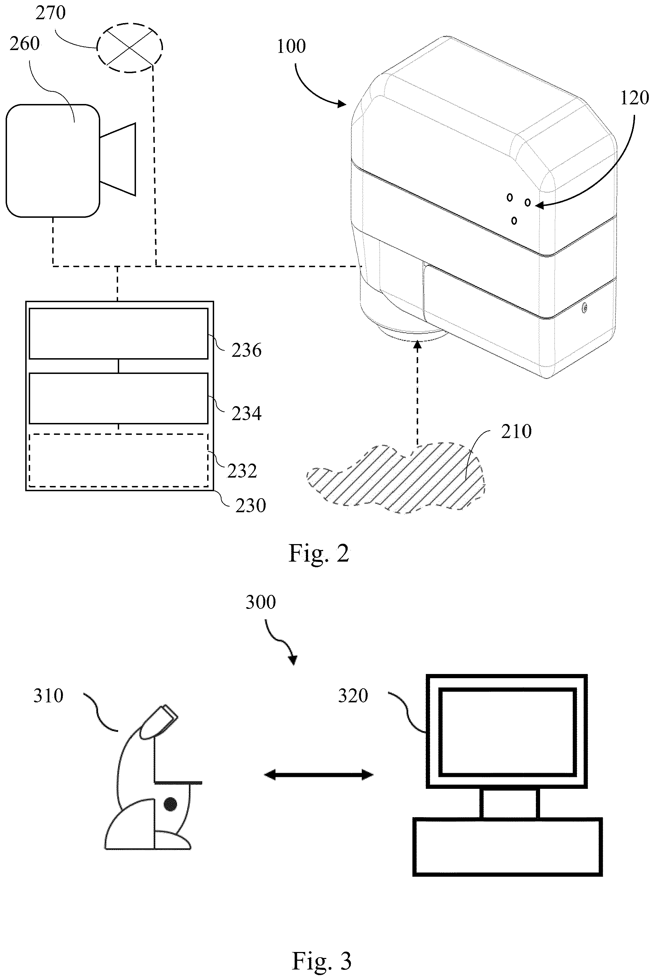

More details and aspects are mentioned in connection with the examples described below. The example shown in Fig. 1 may comprise one or more optional or additional features corresponding to one or more aspects mentioned in connection with the proposed concept or one or more examples described below (e.g., Fig. 2 - 3).

Fig. 2 shows an example of an optical imaging system comprising a housing 100 for a microscope as described with reference to Fig. 1. The housing 100 for the microscope may comprise a tracer 120. Further, Fig. 2 shows an apparatus 230. The apparatus 230 is tasked with controlling various aspects for tracking a location and/or orientation of the tracer 120 of the housing 100, e.g., controlling the camera 260 or the optional illumination source 270. Consequently, the apparatus 230 may be implemented as a computer system, which interfaces with the various components of the optical imaging system 200, e.g., the tracer 220. The apparatus 230 may control a tracker device to track the position of the housing 100 of the microscope relative to the sample 210. The tracker device may comprise the camera 260 configured to acquire image data for determining a location and/or an orientation of the tracer 120 and the optional illumination source 270. In comparison to tracker devices known in the prior art, the tracer 120 is part of the housing 100 and not external to the housing 100. The apparatus 230 may be part of the optical imaging system 200. Alternatively, the apparatus 230 may be communicatively coupled to the optical imaging system 200, e.g., may be part of the camera 260. For example, the apparatus 230 may be a ready-to-use-module, which could be connected to the optical imaging system 200 and/or the camera 260.

The apparatus 230 comprises, as shown in Fig. 2, one or more processors 234 and one or more storage devices 236. Optionally, the apparatus 230 further comprises one or more interfaces 232. The one or more processors 234 are coupled to the one or more storage devices 236 and to the optional one or more interfaces 232. In general, the functionality of the apparatus 230 may be provided by the one or more processors 234 (e.g., for controlling the tracer 220), in conjunction with the one or more interfaces 232 (for exchanging information, e.g., to turn the tracer 220 on or off) and/or with the one or more storage devices 236 (for storing and/or retrieving information).

As described with reference to Fig. 1, the tracer 120 may comprise passive parts such like reflective markers and/or active parts such like a light emitting diode. When the tracer 120 comprises active parts, the active part may be controlled by the apparatus 130. When the tracer 120 comprises passive parts, the illumination source 270 may be controlled by the apparatus to radiate light towards the tracer 120.

More details and aspects are mentioned in connection with the examples described above and/or below. The example shown in Fig. 2 may comprise one or more optional or additional features corresponding to one or more aspects mentioned in connection with the proposed concept or one or more examples described above (e.g., Fig. 1) and/or below (e.g., Fig. 3).

Examples provide a microscope comprising a housing as described with reference to Fig. 1 and an optical component. The optical component is housed by the housing. In an example, the microscope may be a surgical microscope. The optical component may be a lens, a prism, and/or mirror, for example. The apparatus as described above may be part of the microscope.

Examples provide an optical imaging system 300 comprising a housing as described with reference to Fig. 1 or a microscope as described above. There are a variety of different types of optical imaging systems. If the optical imaging system 300 is used in the medical or biological fields, a sample may be a sample of organic tissue, e.g., arranged within a petri dish or present in a part of a body of a patient. In some examples of the present disclosure the optical imaging system 300 may be a surgical optical imaging system, e.g., an optical imaging system that is to be used during a surgical procedure, such as an oncological surgical procedure or during tumor surgery. However, the proposed concept may also be applied to other types of microscopy, e.g., microscopy in a laboratory or microscopy for the purpose of material inspection. Fig. 3 shows a schematic illustration of a system 300, e.g., an optical imaging system, comprising a housing as described with reference to Fig. 1 or a microscope as described above. The system 300 comprises a microscope 310 and a computer system 320. The microscope 310 is configured to take images and is connected to the computer system 320. The computer system 320 is configured to execute at least a part of a method described herein. For example, the computer system 320 may be part of an IGS measurement system. The computer system 320 may be configured to execute a machine learning algorithm. The computer system 320 and microscope 310 may be separate entities but can also be integrated together in one common housing. The computer system 320 may be part of a central processing system of the microscope 310 and/or the computer system 320 may be part of a subcomponent of the microscope 310, such as a sensor, an actor, a camera or an illumination unit, etc. of the microscope 310.

The computer system 320 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

More details and aspects are mentioned in connection with the examples described above. The example shown in Fig. 3 may comprise one or more optional or additional features corresponding to one or more aspects mentioned in connection with the proposed concept or one or more examples described above (e.g., Fig. 1 -2).

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 housing
110 outer shell
120, 122, 124 tracer
130, 132,134 side wall
210 sample
230 apparatus
234 processor
236 storage device
260 camera
270 illumination source
300 system
310 microscope
320 computer system

## Claims

1. A housing (100) for a microscope, comprising:
an outer shell (110), wherein the outer shell (110) forms a cavity for arranging an optical component of the microscope; and
a tracer (120) arranged in the outer shell (110).

2. The housing (100) according to claim 1, wherein
the tracer (120) is arranged in a side wall (130) of the outer shell (110).

3. The housing (100) according to any one of the preceding claims, wherein
the side wall (130) has a wall surface.

4. The housing (100) according to any one of claims 2-3, further comprising
a plurality of tracers (120; 122; 124) arranged in a plurality of side walls (130; 132; 134) of the outer shell (110).

5. The housing (100) according to any claim 4, wherein
a further tracer (122; 124) of the plurality of tracers is arranged in a second side wall (132; 134) adjacent to the side wall (130).

6. The housing (100) according to any one of claims 3-5, wherein
an opposing tracer of the plurality of tracers (120; 122; 124) is arranged in an opposing side wall opposite the side wall (120).

7. The housing (100) according to any one of the preceding claims, wherein
the tracer (120) comprises at least one of a light source and a reflective marker.

8. The housing (100) according to any one of the preceding claims, wherein
the tracer (120) is configured to allow a determination of a position of the housing (100) in three dimensions.

9. The housing (100) according to any one of the preceding claims, wherein
the cavity is completely surrounded by the outer shell (110).

10. A microscope, comprising:
a housing (100) according to any one of the preceding claims; and
an optical component, wherein the optical component is housed by the housing (100).

11. The microscope according to claim 10, wherein
the microscope is a surgical microscope.

12. An optical imaging system (100), comprising
a housing (100) according to any one of claims 1-9 or a microscope according to claim 10 or 11.
